**Europäisches Patentamt**

**European Patent Office**

**Office européen des brevets**

(11) Publication number : **0 549 264 A1**

# (12) EUROPEAN PATENT APPLICATION

(21) Application number : **92311541.4**

(51) Int. Cl.⁵ : **C12N 9/18**, C12P 35/00

(22) Date of filing : **17.12.92**

The applicant has filed a statement in accordance with Rule 28 (4) EPC (issue of a sample only to an expert). Accession number(s) of the deposit(s): NRRL B-18945

The microorganism(s) has (have) been deposited with Agricultural Research Culture Collection (NRRL). under number(s) NRRL B-18945

(30) Priority : **20.12.91 US 811387**

(43) Date of publication of application :
**30.06.93 Bulletin 93/26**

(84) Designated Contracting States :
**AT BE CH DE DK ES FR GB GR IE IT LI LU NL PT SE**

(71) Applicant : **OKLAHOMA STATE UNIVERSITY**
**101 Whitehurst**
**Oklahoma, Oklahoma (US)**

(72) Inventor : **Usui, Shigeyuki**
**3-59, Nishizaki-cho**
**Ogaki 503 (JP)**
Inventor : **Yu, Chang-An**
**4604 Fairfield Drive**
**Stillwater, Oklahoma 74074 (JP)**

(74) Representative : **Hudson, Christopher Mark**
**Erl Wood Manor**
**Windlesham Surrey GU20 6PH (GB)**
**Declaration under Rule 28(4) EPC (expert solution)**

(54) **Purified para-nitrobenzyl esterase from bacillus.**

(57)   The invention provides purified PNB esterase from a microorganism of the genus *Bacillus*. The enzyme is a monomer having a molecular weight of about 54,000 daltons. This PNB esterase catalyzes the deesterification of PNB-esters of cephalosporin and 1-carbacephalosporin compounds to the free acid form. The invention also provides a process for purifying the enzyme from a *Bacillus* species. This process comprises a combination of ammonium sulfate fractionation, pH treatment, anion-exchange chromatography, gel filtration, adsorption-desorption chromatography, and affinity chromatography.

EP 0 549 264 A1

## BACKGROUND OF THE INVENTION

This invention relates to the field of enzyme technology. In particular, it relates to the enzyme *p*-nitrobenzyl-esterase (PNB esterase) from the genus *Bacillus* and to a process for preparing this enzyme in purified form. Esters of cephalosporins and 1-carbacephalosporins are commonly employed intermediates in the synthesis of these antibiotics in free acid form. The preparation of cephalosporins is taught broadly by Chauvette, U.S. Pat. No. 4,064,343. The preparation of 1-carbacephalosporins is taught broadly by Christensen et *al.*, in U.S. Pat. No. 4,226,866, Munroe, in U.S. Pat. No. 4,791,106, and Hirata et *al.*, U.S. Pat. No. 4,708,956.

The PNB-ester function is generally employed to block or protect the acidic carboxylic acid function in cephalosporins and 1-carbacephalosporins while reactions at other sites of the molecules are carried out. For example, Garbrecht, U.S. Pat. No. 3,632,850, describes the use of the *p*-nitrobenzyl ester group in the synthesis of cephalexin. In the first step of the synthesis, this ester is cleaved via hydogenolysis under acidic conditions. In U.S. Pat. No. 3,781,282, Garbrecht describes the de-esterification of *p*-nitrobenzyl esters of cephalosporins with zinc and acid in an amide-type solvent, such as dimethylformamide. Jackson, U.S. Pat. No. 3,799,924, describes the removal of the *p*-nitrobenzyl ester group of cephalosporin esters by treatment with sodium or potassium dithionite at a pH above about 7. Hatfield, U.S. Pat. No. 4,091,214, describes a process for de-esterifying esters of cephalosporin compounds which comprises a reductive cleavage employing an inert solvent with zinc and an α-hydroxycarboxylic acid. Hirata et *al.*, U.S. Pat. No. 4,708,956, describes methods for removal of *para*-nitrobezyl protecting groups from 1-carbacephalosporin compounds. Attendant with these de-esterification procedures is the high cost of recycling solvents and the potential problem of pollution caused by organic solvents.

An alternative, enzymatic method for removal of PNB blocking groups used in the synthesis of cephalosporin and carbacephalosporin antibiotics would have distinct advantages. Such a reaction, proceeding under mild conditions, could be completed in an aqueous reaction mixture without the use of organic solvents and metallic catalysts. Brannon, U.S. Pat. No. 3,972,774, described a process for the removal of the PNB-ester from cephalosporin esters which comprises reacting the ester with a crude preparation derived from a microorganism of the genus *Bacillus*. However, the enzyme responsible for this cleavage was not isolated nor was it determined whether a single enzyme was responsible for the cleavage.

Because of the importance of the *p*-nitrobenzyl esters of cephalosporin and 1-carbacephalosporin antibiotics in the synthesis of these antibiotics in the free acid form, improved methods for the removal of this ester group continue to be the subject of investigation.

Figure 1 is the semilogarithimic plot of the molecular weight of protein standards and PNB esterase against mobility by SDS-PAGE.

Figure 2 is a restriction enzyme site and function map of plasmid pNBE1.

Figure 3 is a restriction enzyme site and function map of plasmid pNB106R.

The invention provides purified PNB esterase from a microorganism of the genus *Bacillus*. The enzyme is a monomer having a molecular weight of about 54,000 daltons. This PNB esterase catalyzes the de-esterification of PNB-esters of cephalosporin and 1-carbacephalosporin compounds to the free acid form. The invention also provides a process for purifying the enzyme from a *Bacillus* species. This process comprises a combination of ammonium sulfate fractionation, pH treatment, anion-exchange chromatography, gel filtration, adsorption-desorption chromatography, and affinity chromatography.

This invention provides the enzyme PNB esterase from a microorganism of the genus *Bacillus* in substantially purified form. As used herein, substantially purified form means greater than ten fold higher specific activity than the *Bacillus* cell extracts. It further provides a method of isolating the purified enzyme which is useful in a method of producing the free acid form of a cephalosporin or 1-carbacephalosporin from the PNB-ester form. The enzyme is a monomeric protein having a molecular weight of about 54,000 Daltons as determined by SDS-polyacrylamide gel electrophoresis (SDS-PAGE). The amino acid composition of the enzyme is shown in TABLE 1. The amino acid composition was determined by conventional as well as high performance liquid chromatography as described below. The PNB esterase provided herein is stable from about 4-50°C and at a pH from about 5.5-8.5.

This invention also provides an enzymatic cleavage process for the removal of the p-nitrobenzyl group from cephalosporin esters and 1-carbacephalosporin esters to provide the corresponding cephalosporin acid and 1-carbacephalosporin acid. The process comprises reacting a cephalosporin ester or 1-carbacephalosporin ester preferably in an aqueous medium, at 25° to 40°C, with the substantially purified PNB esterase of this invention, and recovering the resulting cephalosporin acid or 1-carbacephalosporin acid.

The process has particular value in the preparation of cefaclor and loracarbef when the 4-carboxylic group of these antibiotics is protected during synthesis by a para-nitrobenzyl group.

The loracarbef nucleus free acid is a substrate for penicillin G amidase in a reaction in which phenylglycine methyl ester reacts with this compound to form loracarbef and methanol. Likewise, penicillin G amidase catalyzes conversion of cefaclor nucleus free acid or cephalexin nucleus free acid in conjunction with phenylglycine methyl ester to cefaclor or cephalexin and methanol.

TABLE 1

| amino acid | number of residues per 54,000 Daltons |
|---|---|
| Asx (Asp + Asn) | 34 |
| Thr | 27 |
| Ser | 25 |
| Glx (Glu + Gln) | 49 |
| Pro | 45 |
| Gly | 28 |
| Ala | 41 |
| Val | 21 |
| Cys | N.D. |
| Met | 8 |
| Ile | 17 |
| Leu | 42 |
| Tyr | 14 |
| Phe | 19 |
| Lys | 20 |
| His | 11 |
| Arg | 13 |
| Trp | N.D. |

The 22-residue amino-terminal amino acid sequence of PNB esterase was determined by automated Edman degradation with a gas-phase sequencer. The following sequence was obtained: Met Thr His Gln Ile Val Thr Thr Gln Tyr Gly Lys Val Lys Gly Thr Thr Glu Asn Gly Val His (SEQ ID. NO: 1).

The following internal amino acid sequences were obtained for protease-digested PNB esterase: Glu Asn Ile Phe Gln Leu Phe Phe Gln Pro Ala Leu Asp (SEQ ID. NO: 2); Ala Phe His Ala Leu Glu Leu Pro Phe Val Phe Gly Asn Leu Asp Gly Leu Glu Xaa Met Ala Lys (SEQ ID. NO: 3); Gly Ile Pro Tyr Ala Lys Pro Pro Val Gly Gln Trp Trp Phe Lys (SEQ ID. NO: 4).

The effect on the PNB esterase activity of PNB esterase by various compounds recognized as inhibitors of other enzymes was measured according to the method of Example 1D. The results are shown in TABLE 2. The PNB esterase activity is strongly inhibited by three serine-specific compounds -(phenylmethylsulfonylfluoride, diethyl *p*-nitrophenylphosphate, and diisopropyl fluorophosphate), and one histidine specific compound -(diethylpyrocarbonate). The enzyme was moderately inhibited by an arginine-specific compound (phenylglyoxal), a carboxyl-directed compound (dicyclohexyl carbodiimide) and two sulfhydryl-directed compounds (5,5'-dithiobis(2-nitrobenzoic acid and mercury chloride). The PNB esterase was moderately inhibited by N-benzoyl phenylalanine naptylamide, a substrate of trypsin.

TABLE 2

| Effect of Inhibitors on PNB Esterase | |
|---|---|
| Compound (1 mM) | Inhibition (%) |
| No Addition | 0 |
| Phenylmethylsulfonylfluoride | 100 |
| Diethyl $p$-nitrophenylphosphate | 100 |
| Diisopropyl fluorophosphate | 100 |
| Diethylpyrocarbonate | 100 |
| Dicyclohexyl carbodiimide | 78 |
| Phenylglyoxal | 91 |
| 5,5'-Dithiobis(2-nitrobenzoic acid) | 69 |
| N-benzoyl phenylalanine naptylamide | 66 |
| $HgCl_2$ | 100 |
| FeSO4 | 100 |
| $Zn(OCOCH_3)_2$ | 97 |
| 2-Mercaptoethanol | 0 |
| EDTA | 0 |

Table 3 provides an indication of the substrate specificity of the PNB esterase.

TABLE 3

Substrate Specificity of PNB Esterase

| Substrate | Concentration (nM) | Relative Activity (%) | Km (mM) | Vmax (Unit/mg) |
|---|---|---|---|---|
| Loracarbef-PNB (oxalate) | 0.5 | 100 | 1.1 | 12.8 |
| Loracarbef-PNB (tosylate) | 0.5 | 41 | 1.0 | 4.4 |
| Loracarbef nucleus-PNB | 1.0 | 52 | 1.1 | 6.8 |
| Loracarbef nucleus-PNB | 0.5 | 35 | | |
| Cefaclor nucleus-PNB | 0.5 | 16 | 1.1 | 2.0 |
| Cephalexin-PNB (tosylate) | 0.5 | 12 | 1.3 | 1.7 |
| p-nitrophenyl acetate | 1.6 | 6300 | 0.56 | 381.7 |
| p-nitrobenzyl acetate | 5.0 | 268 | | |
| benzyl acetate | 2.0 | 57 | | |
| α-naphtyl acetate | 2.0 | 57 | | |
| N-benzoyl-DL-phenylalanine-β-naptylester | 0.5 | 43 | | |

The enzyme provided by this invention may be obtained from microorganisms of the genus *Bacillus*. Many of these organisms are known and available and include, for example, *Bacillus subtilis* NRRL B558, *Bacillus circulons* ATCC 966, *Bacillus cereus* NRRL B569, *Bacillus lichenformis* ATCC 7072, *Bacillus cereus* ATCC 9634, *Bacillus subtilis* NRRL 1471, *Bacillus subtilis* NRRL B-8097, and *Bacillus subtilis* ATCC 6633. A particular organism from which the PNB esterase has been isolated is *Bacillus subtilis* NRRL B-8097. A culture of this microorganism has been deposited in the permanent culture collection of the Northern Regional Research Laboratory, U.S. Department of Agriculture Service, Peoria, IL 61604, and is available under accession number B-8097.

The process for purifying PNB esterase described hereinafter is applicable to *Bacillus* organisms which contain the enzyme in varying concentrations. The amount of enzyme in the starting microorganism is not limiting if the enzyme is present in any reasonable amount.

A crude enzyme preparation is obtained from whole cells of the producing organism by harvesting the cells from culture medium in a conventional manner. The cells are suspended in Buffer A (10 mM potassium phosphate, pH 7.0; 1 mM 2-mercaptoethanol (2-ME); and 0.5 mM EDTA). The suspension is sonicated at 0°C, centrifuged and the cell free extract is collected.

The process for preparing the PNB esterase in substantially pure form comprises the following steps:

a) adding ammonium sulfate at about pH 7 to an aqueous cell-free extract containing PNB esterase to achieve a concentration of ammonium sulfate between about 45% and 80% of saturation, and collecting the resulting precipitate;

b) dialyzing a solution of the precipitate buffered at about pH 7 in the presence of a reducing agent;

c) acidifying the dialysate to about pH 5, removing the precipitates and adjusting the dialysate to about pH 8.5;

d) chromatographing the dialysate over a weak anionic exchange resin and eluting the PNB esterase from said resin with a linear gradient of 0.05 M to 0.3 M NaCl in a buffer of about pH 7 in the presence of a reducing agent;

e) pooling and concentrating the PNB esterase-containing eluate of step d;

f) filtering the concentrate of step e through a polysaccharide-type gel at about pH 8.0, in the presence of a reducing agent;

g) pooling and concentrating the PNB esterase-containing eluate of step f, and dialyzing a solution of the concentrate buffered at pH 8.5, in the presence of a reducing agent;

h) chromatographing the concentrate of step g over an anionic exchange resin at about pH 8.5 and eluting the PNB esterase from said resin with a linear gradient of 0.1 M to 0.3 M NaCl in a buffer of about pH 6, in the presence of a reducing agent;

i) pooling and concentrating the PNB esterase-containing eluate of step h and dialyzing a solution of the concentrate buffered at about pH 5.0, in the presence of a reducing agent, and collecting the dialysate;

j) chromatographing the dialysate over calcium phosphate-cellulose at about pH 6, and eluting the PNB esterase therefrom with a linear gradient of 0.01 to 0.10 M potassium phosphate at about pH 7, in the presence of a reducing agent;

k) pooling and concentrating the PNB esterase-containing eluate of step j, dialyzing a solution of the concentrate buffered at about pH 8 in the presence of a reducing agent, and collecting the dialysate;

l) chromatographing the dialysate over a p-aminobenzamidine-agarose at about pH 8, and eluting the PNB esterase therefrom with a linear gradient of 0 to 0.3 M NaCl at about pH 8, in the presence of a reducing agent;

m) pooling and concentrating the PNB esterase-containing eluate of step 1;
wherein steps a-m are carried out at a temperature between about 0°C and 4°C.

A key feature of the process comprises the use of a series of buffers and chromatographic resins which allow separation of the PNB esterase from other proteins while maintaining the activity of the enzyme.

In the above-described purification process reducing agents other that 2-ME may be used in the buffer. For example, dithiothreitol, N-acetyl-L-cysteine and like sulfhydryl reagents may be used. 2-ME is preferred.

The ultrafiltration of the chromatographic eluates is carried out in conventional manner for the purpose of reducing the volume of the eluates for subsequent steps in the process.

The amino-terminal and internal amino acid sequences of PNB esterase provided herein, are useful for the purpose of cloning the gene that codes for PNB esterase. Cloning and expression of the PNB esterase gene using recombinant DNA technology has the potential of providing greater amounts of the enzyme.

The present invention provides a process for preparing PNB esterase in substantially purified form from a recombinant host cell which is capable of expressing PNB esterase, which comprises the steps:

a) adding ammonium sulfate at about pH 7 to an aqueous cell-free extract containing PNB esterase to achieve a concentration of ammonium sulfate between about 45% and 85% of saturation, and collecting the resulting precipitate;

b) dialyzing a solution of the precipitate buffered at about pH 7 in the presence of a reducing agent;

c) acidifying the dialysate to about pH 5, removing the precipitates and adjusting the dialysate to about pH 8.5;

d) chromatographing the dialysate over a weak anionic exchange resin and eluting the PNB esterase from said resin with a linear gradient of 0.05 M to 0.3 M NaCl in a buffer of about pH 7 in the presence of a reducing agent;

e) pooling and concentrating the PNB esterase-containing eluate of step d;

f) chromatographing the dialysate over a p-aminobenzamidine-agarose at about pH 8, and eluting the PNB esterase therefrom with a linear gradient of 0 to 0.30 M NaCl at about pH 8, in the presence of a reducing agent;

g) pooling and concentrating the PNB esterase-containing eluate of step f, and dialyzing a solution of the concentrate buffered at pH 8.5 in the presence of a reducing agent

h) chromatographing the concentrate of step g over an anionic exchange resin at about pH 8.5 and eluting the PNB esterase from said resin with a linear gradient of 0.1 M to 0.3 M NaCl in a buffer of about pH 6, in the presence of a reducing agent;

i) pooling and concentrating the PNB esterase-containing eluate of step h and dialyzing a solution of the concentrate buffered at about pH 5.0, in the presence of a reducing agent, and collecting the dialysate;

wherein steps a-i are carried out at a temperature between 0°C and 4°C.

The following Examples are intended to assist in the further understanding of the invention. Particular materials employed, species, and conditions are intended to be further illustrative of the invention and not limiting the reasonable scope thereof.

## EXAMPLE 1

Isolation and Purification of PNB Esterase from *Bacillus subtilis*

### A. Preparation of Cell-Free Extract

A culture of *Bacillus subtilis* NRRL B-8079 was harvested and frozen by conventional methods. The frozen *B. subtilis* cells (760 g) were thawed and homogenized in 2 liters of Buffer A. The cell free extract was obtained by centrifugation at 24,000 x g for 30 minutes. Protamine sulfate was added to the extract at a final concentration of 2.0 mg/ml and the mixture was stirred for 1 hour. The precipitates formed were removed by centrifugation. The supernatant was used as the cell-free extract in the following isolation procedure.

### B. Isolation of Crude PNB esterase

The extract was subjected to ammonium sulfate fractionation. The precipitates formed between 45-80% ammonium sulfate saturation were collected by centrifugation and dissolved in Buffer A, and then dialyzed overnight against the same buffer. The dialyzed sample was acidified to pH 5.0 with 1 N acetic acid, incubated for 10 minutes and subjected to centrifugation to remove the precipitates. The supernatant contained the crude PNB esterase.

### C. Purification of PNB esterase

The supernatant was adjusted to pH 8.5 with 2 N NH$_4$OH an applied onto a weak anionic resin (DE52 column, 3.7 x 35 cm; Pharmacia, Inc., Piscataway, NJ 08854) equilibrated with Buffer B (10 mM Tris-HCl, pH 8.5; 50 mM NaCl; 1 mM 2-ME; and 0.5 mM EDTA). The column was washed with 350 ml of Buffer B and again with 1,750 ml of Buffer C (10 mM Tris-HCl, pH 7.0; 50 mM NaCl; 1 mM 2-ME; 0.5 mM EDTA). The PNB esterase was eluted with a 3,500 ml gradient of 50-300 mM NaCl in Buffer C. Fractions of the PNB esterase were pooled and concentrated by ultrafiltration with an AMICON PM-10 membrane (Amicon, Danvers, MA 01923). The concentrated crude PNB esterase solution was subjected to gel filtration employing a polysaccharide-type gel (Sephacryl S-200 HR column, 5.0 x 95 cm; Pharmacia, Inc.) equilibrated with Buffer D (10 mM Tris-HCl, pH 8.0; 1 mM 2-ME; 0.5 mM EDTA). The fractions containing enzyme activity were combined, concentrated by ultrafiltration and dialyzed overnight against Buffer B. The dialyzed solution was applied onto an anionic exchange resin, (Q-Sepharose column, 2.6 x 20 cm; Pharmacia Inc,) equilibrated with Buffer B. The column was washed with 100 ml of Buffer B and subsequently with 500 ml of Buffer E (10 mM MES-NaOH, pH 6.0; 100 mM NaCl; 1 mM 2-ME; 0.5 mM EDTA). The PNB esterase was eluted with a 1,500 ml linear gradient of 100-300 mM NaCl in Buffer E. The fractions containing the PNB esterase were combined, concentrated by ultrafiltration, and dialyzed overnight against 10 mM sodium acetate, pH 5.0, containing 1 mM 2-ME and 0.5 mM ethylenediamine tetraacetic acid.

After removing the precipitate by centrifugation, the enzyme solution was loaded onto a calcium phosphate-cellulose column (1.6 x 20 cm) equilibrated with a buffer containing 10 mM sodium acetate, pH 5.0, containing 1 mM 2-ME and 0.5 mM EDTA. The finely-divided calcium phosphate used can be prepared according to Jenner, U.S. Pat. No. 3,737,516. After the column was washed with 250 ml of the same buffer, the PNB esterase was eluted with a 250 ml linear gradient of 10-50 mM potassium phosphate, pH 7.0, containing 1 mM 2-ME and 0.5 mM EDTA. The fractions containing the PNB esterase activity were pooled, concentrated by ultrafiltration, and then dialyzed overnight against Buffer D.

The dialyzed enzyme solution was applied onto a p-aminobenzamidine-agarose column (1.0 x 20 cm; Pharmacia Inc.) equilibrated with Buffer D. After washing the column with 50 ml of Buffer D, the PNB esterase was eluted with 100 ml linear gradient of 0-300 mM NaCl in Buffer D. The fractions with the PNB esterase activity eluted approximately between 160 and 220 mM NaCl in Buffer D were pooled, concentrated by ultrafiltration, and represented the purified enzyme.

All steps of the above-described purification were performed at temperatures between 0-4°C. The progress of the purification was followed by assaying the material for PNB esterase activity obtained in each step of the isolation and purification. TABLE 4 shows the results obtained after each step.

## TABLE 4

### Purification of PNB Esterase from *Bacillus subtilis* Substrate

| Step | Protein [1] (mg) | LORACARBEF Activity (U) | LORACARBEF Specific activity (U/mg) | CEPHALEXIN Activity (U) | CEPHALEXIN Specific activity (U/mg) |
|---|---|---|---|---|---|
| Cell-free extract | 41400 | 95.2 | 2.3 | 9.81 | 0.24 |
| 45-80% AmSO₄ fraction | 18400 | 49.6 | 2.7 | 5.64 | 0.31 |
| pH treatment | 11000 | 44.2 | 4.0 | 5.18 | 0.47 |
| DE52 eluate | 2300 | 52.0 | 22.6 | 6.15 | 2.67 |
| Sephacryl S-200 eluate | 985 | 47.5 | 48.2 | 5.98 | 6.07 |
| Q-Sepharose eluate | 16 | 20.5 | 1280 | 1.72 | 108 |
| Calcium phosphate-cellulose eluate | 6.9 | 11.7 | 1690 | 1.04 | 150 |
| p-amino-benzamidine agarose eluate | 4.0 | 8.0 | 2030 | 0.87 | 220 |

[1] The total protein was determined by the method of Lowery, O.H., et al., J. Biol. Chem. 193:265 (1951).

D. Assay Method for PNB Esterase Activity

A 1 ml reaction mixture containing 5 μmoles Bis-Tris-Propane-HCl, pH 6.5, 0.5 μmole substrate (loracarbef PNB ester, or cefaclor PNB ester), and appropriate amounts of enzyme solution was incubated at 37°C in a constant-temperature shaker for 30 minutes The reaction was stopped by the addition of an equal volume of acetonitrile. The mixture was then centrifuged to remove the protein and the supernatant solution was analyzed by high performance liquid chromatography (HPLC) for product formation and substrate disappearance. The HPLC was performed in a C-18 reverse-phase column (Nova-Pak $C_{18}$ radial-pak cartridge (8 X 100 mm), Millipore Bedford, MA) with a linear gradient formed by Buffer A containing 80% of 1 mM Triethylamine-HCl, pH 2.5 and 20% methanol and Buffer B containing methanol, at a flow rate of 1 ml/minutes

The HPLC system used was a Varian HPLC system including a Vista 5560 unit (Varian Associates, Sugar Land TX) and a model 230-401 auto-sampling injector (Gilson Medical Electronics, Middleton, WI.). The PNB esterase activity was determined by monitoring product formation at 254 nm.

Taking advantage of the absorption change in the hydrolysis of *p*-nitrophenylacetate to *p*-nitrophenyl alcohol and acetate, a spectrophotometric assay for PNB esterase was developed. An enzyme fraction showing activity in this assay may not necessarily have the PNB esterase activity, but the PNB esterase will show activity in this assay. For the purified PNB esterase, the results of this assay correlated well with that of the HPLC assay. Thus, this assay is valuable in the purification of the enzyme, where large numbers of assays are generally involved. This assay was carried out at room temperature in a 1 ml assay mixture, containing 100 μM Tris-HCl (pH 7.0), 1.6 μM *p*-nitrophenylacetate and 1-20 μl enzyme solution. The activity was followed by measuring the absorption change at 405 nm in either a Cary spectrophotometer, model 219 or Beckman DU-50.

E. Molecular Weight Determination

The molecular weight determination of the PNB esterase by sodium dodecylsulfate polyacrylamide gel electrophoresis (SDS-PAGE) was carried out as follows. The purified PNB esterase, 2 μg, treated with 2% SDS and 5% 2-ME, was applied to the gel. The electrophoresis was performed with protein standards of Laemmli, U.K., 1970, nature 227:680-682. The protein standards used were (1) phosphorylase B (molecular weight (M.W.)=92,500); (2) bovine serum albumin (M.W.=66,200); (3) ovalbumin (M.W.=45,000); (4) carbonic anhydrase (M.W.=31,000); (5) soybean trypsin inhibitor (M.W.=21,500); and (6) lysozyme (M.W.=14,400).

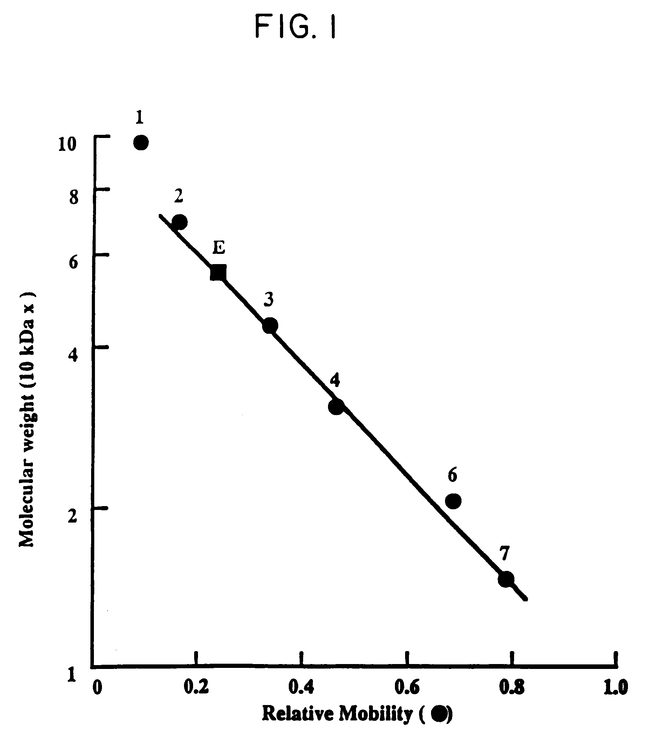

Figure 1 of the drawings is a semilogarithmic plot of molecular weight against mobility on the gel. The numbers in the plot indicate the protein standards used as shown above in parenthesis. E designates PNB esterase.

F. Amino Acid Sequence and Composition

Amino acids were analyzed by the methods of Heinrikson and Meredith (1984) *Anal. Biochem.* 136, 65-74, and Bidlingmeyer *et al.*, (1984) *J. Chromatog.* 336:93-104, with HPLC reverse phase after derivatization with phenylisothiocyanate (PITC) to phenylthiocarbamoyl amino acids (PTC-amino acids).

The purified PNB esterase was extensively dialyzed against water and lyophilized in acid-washed tubes and then hydrolyzed with 6 N HCl at 110° for 24 hours. The hydrolysate was reacted with PITC and the PTC-amino acids formed were quantitated by HPLC with external amino acid standards using an Ultrosphere-ODS column (0.46 cm X 25 cm) (Beckman). The PTC-amino acids were eluted with the gradient of solvents (A) 50 mM ammonium acetate, pH 6.0, and (B) a mixture of acetonitrile-methanol-0.22 M ammonium acetate, pH 6.0, (44:10;46). Additionally, the amino acid composition of the PNB esterase was analyzed by conventional ninhydrin procedure.

The amino acid sequence analysis was carried out by Edman degradation using a model 470A gas phase protein sequencer with on-line detection of the released amino acid phenylthiohydantoin derivatives (PTH-amino acids). The derivatives were detected by a model 120A PTH-amino acid analyzer of Applied Biosystems(Foster City, CA), Applied Biosystems Protein Sequencer User Bulletin No. 12 (1985). The purified PNB esterase, which was extensively dialyzed against water and lyophilized, was dissolved in 5% acetonitrile containing 0.1% trifluoroacetic acid and was then adsorbed into the polybrene-coated glass microfiber filter.

EXAMPLE 2

Isolation of PNB Esterase Gene (*pnbA*) from a Genomic Library of *Bacillus subtilis* (NRRL B8079) DNA

A. Description and Genotypes of Strains

*Bacillus subtilis* strain NRRL B8079 was isolated in a screen designed to identify microorganisms capable of removing the *para*-nitrobenzyl ester from cephalosporins (Brannon *et al.*, *J. Antibiotics* XXIX No. 2:121-124 1976). *B. subtilis* NRRL B-8079 has been deposited in the permanent culture collection of the Northern Regional Research Laboratory (NRRL), United States Department of Agriculture Service, Peoria, IL 61604, and is available under accession number B-8079. *Escherichia coli* K12 DH5α™ (MAX Efficiency DH5α™ Compet-

ent Cells; GIBCO BRL, Gaithersburg, MD), which is a recA⁻ strain that has been developed to be highly transformable and provide a stable environment for maintenance of *E. coli* plasmids, was used as host strain for the *B. subtilis* strain NRRL B8079 genomic library (see Example 2J). The *recA+ E. coli* K12 RV308, was used for high-level expression of the cloned PNB esterase gene and is a preferred host for expression of heterologous proteins in *E. coli* on an industrial scale. *E. coli* K12/RV308 has been deposited with the NRRL and is available under accession number B-15624.

## B. Cultivation of Strains

Trypticase®-Soy broth (TSB; Becton Dickinson Microbiology Systems) and Luria broth (L-broth; 10 g Difco Bacto-Tryptone®, 10 g NaCl, and 5 g yeast extract per liter) were used as growth medium for liquid cultures. Cultures on solid medium were grown on L-broth supplemented with 2% w/v agar (L-agar). Antibiotics were added to medium where necessary, at the following concentrations: ampicillin (50 μg/ml), and tetracycline (5 μg/ml). 5-bromo-4-chloro-3-indolyl-D-galactoside (X-gal; Sigma Chemical Co., St. Louis, MO 63178) was added to media at 20 μg/ml to detect β-galactosidase activity.

## C. Transformation of *Escherichia coli* K12 DH5α, *E. coli* K12 RV308, and *E. coli* W ATCC 1105

*Esherichia coli* K12 DH5α competent cells were obtained from BRL and were transformed using the manufacturer's protocol. Alternatively, 50 ml cultures of *Escherichia coli* K12 DH5α, *E. coli* K12 RV308, or *E. coli* W ATCC 1105, were grown in L-broth to an $OD_{590}$ of approximately 0.5 absorbance units, and the cells were collected by centrifugation. The cell pellet was resuspended in 25 ml of cold 100 mM $CaCl_2$ and incubated on ice for 25 minutes. The cells were collected by centrifugation, resuspended in 2.5 ml of cold 100 mM $CaCl_2$ and incubated on ice overnight. Competent cells were used directly or stored at -70°C until ready for use in transformations.

To transform competent **Escherichia coli** cells, one hundred microliters of the competent cell suspension were removed from storage at -70°C and allowed to thaw on ice. Cells were gently mixed with five microliters of a solution of plasmid DNA (1 ng/ml) in a polypropylene tube, and the resulting solution was incubated on ice for 30 minutes. The tube was transferred to a 42°C water bath and incubated without shaking for 45 seconds. After this heat-shock treatment, the tube was placed on ice for 2 minutes. The tube was then removed from the ice and 0.9 ml of S.O.C. medium (2% Bacto-Tryptone®; 0.5% yeast extract; 10 mM NaCl; 2.5 mM KCl; 10 mM $MgCl_2$; 10 mM $MgSO_4$; and 20 mM glucose), pre-incubated to room temperature, was added. The cell suspension was then shaken at 225 rpm for 1 hour at 37°C and aliquots were plated on L-agar containing ampicillin. Putative transformants were picked from plates after incubation at 37°C, and their identity was confirmed by sizing their plasmid DNA using horizontal gel electrophoresis (*Sambrook et al.*, 1989). Plasmid DNA from selected clones was characterized by restriction enzyme analysis.

## D. Plasmid DNA isolation

Plasmid DNA was isolated from *Escherichia coli* strains using a standard alkaline-SDS procedure (Sambrook et al., 1989). Alternatively, plasmid DNA was isolated as follows. A portion of a transformant colony growing on L-agar containing 50 mg/ml ampicillin was transferred to one liter of L-broth containing 50 mg/ml ampicillin and incubated in an air-shaker at 37°C for about 16 hours. Cells were harvested in 500 ml bottles in a Beckman JA-10 rotor at 8000 rpm for 10 minutes at 4°C. The cell pellet was washed with TE, pH 8.0 (10 mM Tris-HCl, 1 mM EDTA), and resuspended in 50 mM Tris-HCl, pH 8.0, 25% sucrose to a total volume of 10 ml. One milliliter of 20 mg/ml lysozyme (Sigma Chemical Co., St. Louis, MO.) in 25 mM Tris-HCl, pH 8.0, was added with stirring, and the mixture was incubated on ice for 30 minutes. Four milliliters of 200 mM EDTA was added, with subsequent incubation on ice for 10 minutes, followed by the addition of 15 ml of Brij/DOC lysing solution (1% Brij 58; 0.4% deoxycholate; 50 mM Tris-HCl, pH 8.0; 60 mM EDTA). Tubes were gently inverted to mix, and incubated on ice for 15-30 minutes. Cell debris was removed by centrifugation at 18,000 rpm in a Beckman JA-20 rotor for 1 hour. Supernatant was decanted yielding approximately 30 ml, to which 150 ml of 10 mg/ml RNAse A (Sigma Chemical Co.) was added. After a 1 hour incubation at 37°C, 150 ml of 10 mg/ml Proteinase K (Boehringer Manneheim) was added followed by another 1 hour incubation at 37°C. DNA was precipitated by the addition of 1/10 volume of 3 M sodium acetate, pH 7.0, followed by 3X volumes of ice cold absolute ethanol. DNA was recovered by centrifugation in a Beckman JA-14 rotor (Beckman Instruments Inc., Fullerton, CA 92634) at 8,000 rpm for 30 minutes. The air dried pellet was resuspended in TE, pH 8.0, to a total volume of 9 ml, to which was added 9 g of cesium chloride (Boehringer Manneheim) and 0.5 ml of 10 mg/ml ethidium bromide. The resulting solution was loaded into two 5.1 ml tubes Quik-Seal (Beckman Instruments Inc.) and centrifuged at 65,000 rpm in a Beckman VTi65.2 ultracentrifuge rotor for 6 hours. The plasmid band was visualized under ultraviolet light and was removed by syringe. The resulting DNA solution was extracted with salt-saturated isopropanol to remove the ethidium bromide, and dialyzed 16 hours against 1000x volumes TE, pH 8.0. DNA solutions were stored at -20°C until needed.

### E. Polymerase chain reaction (PCR) amplifications

Polymerase chain reactions were performed using a DNA Thermal Cycler™ and the Gene-Amp™ reagent kit (Perkin-Elmer Cetus, Norwalk, CT 06859), according to the instructions of the manufacturer. Thirty cycles of amplification were performed, with each cycle consisting of the following incubations: 1 min at 93°C, followed by 2 min at 40°C, followed by 4 min at 72°C. Syntheses were completed by incubation at 72°C for 10 minutes

### F. Nucleotide sequence analysis

Supercoiled plasmid DNA templates for sequencing were purified from alkaline lysates of *Escherichia coli* cultures on Qiagen DNA binding columns (Qiagen, Inc., Chatsworth, CA) according to protocols provided by the manufacturer.. Dideoxynucleotide chain-termination sequencing reactions were performed according to a cycle sequencing protocol (Applied Biosystems, Inc. (ABI), Foster City, CA 94404) with fluorescent dye-labelled dideoxynucleotides, supercoiled plasmid templates, and sequence specific oligonucleotide primers. Sequence reactions were analyzed on an ABI model 373A automated DNA sequencer. Nucleotide sequences were compiled, edited, and analyzed with the GCG computer programs of Devereux et al., 1985, *Nucleic Acids Res.* 12:387-395.

### G. Synthesis and End-labelling of Oligonucleotide Probes

The amino-terminal sequence of PNB esterase purified from *Bacillus subtilis* NRRL B8079 was obtained by subjecting 25 picomoles of purified PNB esterase, specific activity ca. 2.2 U/mg (based on hydrolysis of loracarbef PNB ester to corresponding free acid), to analysis in an automated gas phase sequenator (Hewick *et al.*, 1981, *J. Biol. Chem.* 256:7990; Hunkapiller & Hood, 1978, *Biochemistry* 17:2124). The amino acid sequence of the amino-terminal end of PNB esterase is: Met Thr His Gln Ile Val Thr Thr Tyr Gly Lys Lys Val Lys Gly Thr Gln Glu Asn Gly Val His (SEQ ID NO. 1).

Based on this sequence and the known codon usage for *Bacillus subtilis*, (Harwood et al., *Molecular Biological Methods for Bacillus*, John Wiley and Sons Ltd., West Sussex, England (1990), Two oligonucleotides probes were synthesized on an Applied Biosystems Inc. model 380B DNA synthesizer using β-cyanoethyl phosphoramidite chemistry, according to the manufacturer's instruction. These oligonucleotide probes, called PNB1 and PNB2 are presented below from left to right in the 5' to 3' orientation:

```
PNB1 - ATGACACATC AAATTGTCAC AACATATGGC AAAAAAGTCA A (SEQ ID
NO. 5)


PNB2 - TATGGCAAAA AAGTCAAAGG CACACAAGAA AATGGCGTCC A (SEQ ID
NO. 6).
```

The single stranded DNA segments were first column-purified (Oligonucleotide Purification Cartridges; Applied Biosystems, Inc.) and then end-labelled as follows. Ten picomoles of each probe were added to a 20 µl reaction mixture containing 12 µl [γ-32P] adenosine triphosphate (ATP; 5000 Ci/ml) and 8 units of T4 polynucleotide kinase in kinase buffer (50 mM Tris-HCl, pH 7.6, 10 mM MgCl$_2$). Following a 35 min reaction at 37°C, and a 5 minute incubation at 70°C to inactivate the kinase, the unincorporated [γ-32P] ATP was removed from the reaction mixture using a Sephadex G-50 Nick Column (Pharmacia Inc, Piscataway, NJ 08854).

### H. Isolation of Genomic DNA from *Bacillus subtilis* NRRL B8079

Total DNA was isolated from a two liter culture of *Bacillus subtilis* NRRL B8079 grown to mid-logarithmic phase in TSB at 25°C for 16 hr. Cells were harvested by centrifugation and resuspended in 20 ml of buffer (50 mM Tris-HCl, pH 8.0, 50 mM EDTA). Lysis of cells was accomplished in two steps by: (i) addition of 1 mg/ml lysozyme from egg white; Calbiochem, La Jolla, CA) and incubation of the suspension for 20 min at 37°C, and (ii) addition of 4 ml of lysing buffer (0.5% sodium dodecyl sulfate [SDS], 50 mM Tris pH 7.5, 0.4 M EDTA, 1 mg/ml Proteinase K) and incubation at 50°C for 30 minutes The DNA was purified by two phenol extractions followed by one chloroform extraction, and then recovered by precipitation with ethanol and spooling on a glass rod. RNA was removed from the preparation by gently resuspending the DNA in 40 mM Tris-HCl buffer, pH 7.5, containing 1 mM EDTA and 0.2 mg/ml ribonuclease (from bovine pancreas; Sigma Chemical Co.) and then incubating the preparation at 25°C for 30 minutes Further purification was accomplished by re-extraction with phenol and chloroform, precipitation with ethanol, and resuspension in TE, pH 7.5.

I. Southern Hybridizations of *Eco*RI Restriction Fragments Using Oligonucleotide Probes

Genomic DNA from *Bacillus subtilis* NRRL B8079 was digested with *Eco*RI and subjected to electrophoresis on a 1% agarose-TBE gel (Sambrook *et al.*, 1989). The size-fractionated DNA was then transferred to Hybond-N+ nylon membranes (Amersham, Arlington Heights, IL, U.S.A.) by Southern blotting (Sambrook *et al.*, 1989), and cross-linked to the matrix by treatment with ultraviolet light for 5 minutes After pre-hybridization of the DNA at 70°C for 1-2 hr in hybridization buffer containing 6X SSC (1X SSC, pH 7.0, contains 0.15 M sodium chloride, 0.015 M sodium citrate), 5X Denhardt's solution (1X Denhardt's solution contains 0.2 g/l Ficoll 400, 0.2 g/l polyvinylpyrollidone and 0.2 g/l bovine serum albumin [Fraction V]), 0.25% SDS, and 20 µg/ml calf thymus DNA, fragmented by treatment with ultrasound. Fresh hybridization buffer containing [$^{32}$P]-labelled oligonucleotide (prepared as described in Example 2G) was then added to produce a final probe concentration of 0.5 pmoles/ml. The temperature of the incubation mixture was allowed to fall to 45°C during overnight incubation. The membranes were then washed using conditions of stringency that had been optimized for each probe. Membranes hybridized to PNB1 were washed for 25 min, three times in succession, at 45°C in 4X SSC, 0.25% SDS. Membranes hybridized to PNB2 were washed similarly, with 0.5X SSC, 0.25% SDS. After washing, the membranes were dried at room temperature and exposed to film.

Using the above procedure, a band representing EcoRI fragments of approximately 6 kb in size was shown to hybridize to probes PNB1 and PNB2.

J. Construction of an Enriched Library of *Eco*RI DNA Fragments and Isolation of pNBE1

*Bacillus subtilis* NRRL B8079 genomic DNA was completely digested with *Eco*RI and subjected to electrophoresis in a horizontal 1.2% agarose gel (in 1X TAE buffer containing 40 mM Tris-acetate, 1 mM EDTA; 2V/cm) for 16 hr. The gel was stained in a dilute (1 mg/ml) ethidium bromide solution and the DNA bands visualized under long-wave ultraviolet light. A slice was removed from the region of the gel that corresponded to DNA of approximately 6 kb in size, and that spanned the band that had previously been shown to hybridize to PNB1 and PNB2. Elution of the DNA fragment from the gel slice was performed according to the protocol of Sambrook et al. (1989), with minor adjustments. Briefly, the gel slice was put into a dialysis bag with 200 µl 0.2X TAE buffer, sealed with clips, and electrophoresed in 0.2X TAE buffer for about 3 hours. The contents of the dialysis bag, including a 400 µl wash with 0.2X TAE, were mixed with 2.4 ml of low salt buffer (0.2 M NaCl; 20 mM Tris-HCl, pH 7.5; 1.0 mM EDTA) and loaded onto an ELUTIP-d column (Schleicher & Schuell, Keene, NH) prepared according to the manufacturer's protocol. After washing with low salt buffer the DNA was eluted from the column with two 400 µl volumes of high salt buffer (1.0 M NaCl; 20 mM Tris-HCl, pH 7.5; 1.0 mM EDTA) and precipitated by addition of 800 µl of ice-cold absolute ethanol followed by centrifugation at 14,000 rpm in an Eppendorf 5415C microfuge for 40 minutes. The two air-dried pellets were combined by dissolving in 20 ml TE, pH 7.5, and the solutions were stored at -20°C until ligation. The fragments were ligated into the vector pUC19 (Gibco BRL, Gaithersburg, MD) which was pre-treated by digestion with *Eco*RI followed by removal of the 5' phosphates using calf intestinal alkaline phosphatase (Calbiochem; La Jolla, CA).

The resulting plasmids were used to transform competent *Escherichia coli* K12 DH5α cells, which were then plated onto L-agar plates containing ampicillin and X-Gal. Use of plasmid pUC19 as a vector allowed the specific selection of clones containing insert DNA using blue/white selection. Transformants containing only the pUC19 vector, which includes that portion of the *lacZ* gene that codes for the α-peptide of β-galactosidase, produce active enzyme, detected by cleavage of X-Gal to produce a blue color. In contrast, isolates containing an insert at the *Eco*RI site disrupt the reading frame of the α-peptide, and are unable to convert the X-Gal, and thus remain white on media containing X-Gal. More than one hundred white, ampicillin-resistant colonies were selected and replated on the same agar to test phenotypic stability.

Plasmid DNA was isolated from white colonies and analyzed by gel electrophoresis. Seven colonies containing plasmid DNA of the required 9 kb size (2.686 kb pUC19 DNA plus approximately 6 kb insert DNA) were selected for further study. When tested by Southern hybridizations, using the PNB2 probe, one plasmid, pNBE1, gave a strong hybridization signal. Digestion of plasmid pNBE1 with *Eco*RI yielded two fragments of the predicted size: one corresponding to linearized pUC19 (2.686 kb), and the other, approximately 6 kb in size, corresponding to the insert fragment.

K. Demonstration of the direction of transcription of *pnbA* on plasmid pNBE1

Two PCR amplifications using pNBE1 were performed to determine the direction of transcription of *pnbA*, relative to the *lacZ* gene of pUC19, and the approximate position of the ATG transcription start site in the insert. The first amplification used the PNB2 oligonucleotide probe and the forward (-20) M13 DNA sequencing primer (New England BioLabs). The second amplification also used PNB2, but the second primer was the reverse (-24) M13 sequencing primer. Results showed that only the reaction with the (-20) forward sequencing primer generated an amplified fragment. The size of the amplified fragment was determined to be 2.0 kb. Therefore, the ATG start site of *pnbA* is located approximately 4 (kilobase pairs) downstream of the start of the multi-cloning site of pUC19, and is transcribed in the same direction as the *lacZ* gene.

L. Verification of a functional *pnbA* gene

To determine whether the entire *pnbA* gene was present on pNBE1 and capable of being expressed in *Escherichia coli*, extracts of *E. coli* K12 DH5α cells transformed with plasmid pNBE1 were assayed for esterase activity. Cell extracts were prepared from approximately 75 ml of a culture grown to mid-logarithmic phase in L-broth. Cells were collected by centrifugation for 10 min at 8,000 rpm, washed in 10 mM sodium phosphate buffer, pH 7.0, and centrifuged. The cell pellet was resuspended (1 g cells/4 ml buffer) and disrupted by exposure to four 30 second bursts of ultrasound (Sonicator™ Cell Disruptor Model W185F, Heat Systems-Ultrasonics Inc., Plainview, N.Y.). Cell debris was removed by centrifugation in a microfuge for 20 min at maximum speed. Because purified PNB esterase catalyzes the cleavage of *p*-nitrophenyl acetate, in addition to *p*-nitrobenzyl esters of β-lactam antibiotics, this reaction could conveniently be used for monitoring the formation of *p*-nitrophenol spectrophotometrically. The reaction mixture contained 400 μl of 0.5 M p-nitrophenyl acetate (in 1:99 v/v acetonitrile/water), 600 μl of 167 mM Tris-HCl, pH 7.0, and 1-20 μl of cell free extract. Formation of *p*-nitrophenol was measured spectrophotometrically by monitoring the increase in $OD_{405}$ using a Gilford Response II spectrophotometer. Alternatively, enzyme activity could be demonstrated qualitatively using whole cells by adding a portion of a colony to the reaction mixture using a toothpick. Assay of both whole cells and cell free extracts using these procedures demonstrated that plasmid pNBE1 contained an intact *pnbA* gene, and that the gene was expressed to produce active enzyme. No significant activity was detected in *Escherichia coli* K12 DH5α cells without plasmid, or with *E. coli* K12 DH5α cells carrying other non-hybridizing plasmids from the genomic library.

M. Subcloning of the *pnbA* gene

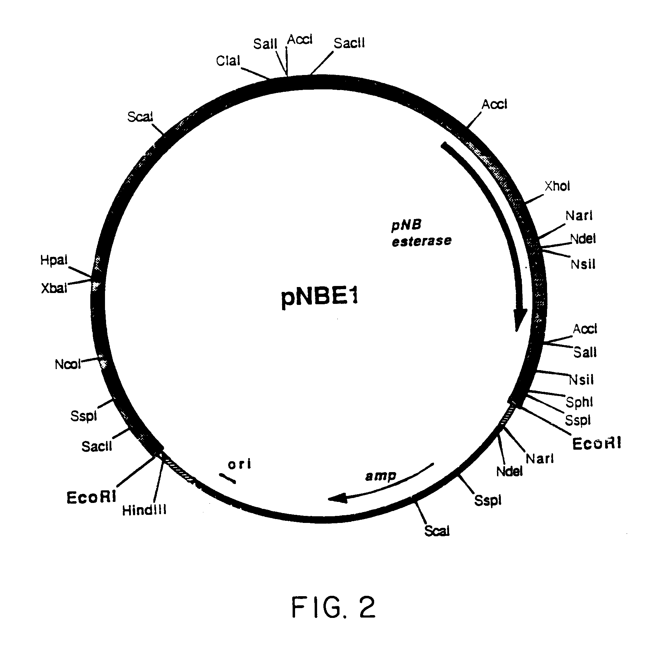

With the information obtained from the PCR experiment described above, and results from single and double restriction enzyme digestions of pNBE1, it was possible to construct a preliminary physical map of the 6.0 kb *Eco*RI fragment. A restriction enzyme site and function map of plasmid pNBE1 is presented in Figure 2. A series of subclones of pNBE1 were then made by partial digestion of the plasmid with *Hpa*II, followed by complete digestion with *Eco*RI, and ligation of the resulting *Hpa*II-*Eco*RI fragments to pUC19 digested with *Acc*I-*Eco*RI. The *Hpa*II site of the insert and the *Acc*I site of pUC19 were not regenerated by this ligation. The resulting plasmids were then transformed into *Escherichia coli* K12 DH5α cells, and ampicillin-resistant colonies were selected and tested for esterase activity. Plasmid DNA was isolated from a number of positive clones and analyzed to determine the size of the *Hpa*II-*Eco*RI inserts. The smallest insert that coded for active enzyme was 2.3 kb. The plasmid vector was designated pNBHpE2.3.

An additional subcloning experiment was performed which indicated that the *sal*I-*Eco*RI fragment at the distal end of the 2.3 kb insert was not required for a functional pnbA gene. pNBHpE2.3 was first digested with *Hind*III and *sal*I, ligated to pUC19 digested with *Hind*III and *sal*I, and then transformed into *Escherichia coli* K12 DH5α cells. The plasmid from this construction, designated pNBH3S1.9, contained a 1.9 kb *Hpa*II-*sal*I insert, and coded for active PNB esterase protein. The insert DNA from this plasmid was subcloned into pBluescript SK(-) (Stratagene, La Jolla, CA) and analyzed to determine the nucleotide sequence.

EXAMPLE 3

High Level Expression of the *pnbA* Gene in *Escherichia coli*

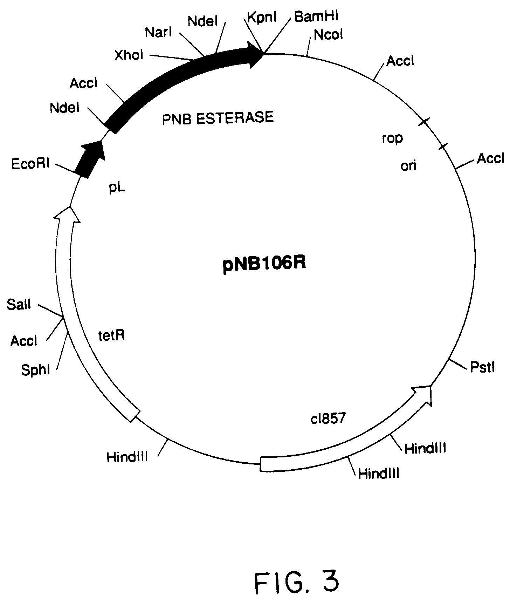

Expression of the cloned PNB esterase gene in *Escherichia coli* was improved by construction of vectors pNB106R. Plasmid pNB106R contains a functional *rop gene*, responsible for control of plasmid copy number (Cesareni et *al.*, 1982, *Proc. Natl. Acad. Sci. USA* 79:6313). A restriction enzyme site and function map of plasmid pNB106R is presented in Figure 3.

A. Construction of *Escherichia coli* K12 DH5α/pNB106R, *Escherichia coli* K12 RV308/pNB106R and *Escherichia coli* W ATCC 11105/pNB106R

Plasmid pNB106R was generated by subcloning the 1.9 kb DNA fragment from plasmid pNBH3S1.9 into the temperature inducible high level expression vector pHKY338 to provide temperature inducible expression of a heterologous gene from a modified bacteriophage lambda pL promoter. *E. coli* K12 RV308/pHKY338 has been deposited in the permanent culture collection of the Northern Regional Research Laboratory (NRRL), United States Department of Agriculture Service, Peoria, IL 61604, on January 31, 1992, and is available under accession number NRRL B-18945. The plasmid contains two regions of DNA of particular importance: (i) a sequence derived from pBR322 which allows replication and maintenance of the plasmid in *Escherichia coli*, and in which the ampicillin resistance gene of pBR322 is replaced by a lambda cI857 repressor gene; and (ii) a sequence in which a modified lambda promoter, pL106 (U.S. Patent Application Serial No. 07/739,280), situated downstream of the tetracycline resistance gene, is immediately adjacent to the open reading frame (ORF) of a kanamycin resistance gene. The promoter controls transcription of that ORF via an "out-of-frame" two cistron assembly (Schoner et *al.*, 1990, *Methods in Enzymology* 185:94). Plasmid pHKY338 was constructed so that the open reading frame of the kanamycin resistance gene can easily be removed by digestion with *Nde*I and *Bam*HI. Any ORF with an *Nde*I restriction site at its ATG transcriptional start codon, and a *Bam*HI compatible restriction site downstream from its potential transcription termination sequences, can be inserted into pHKY338. The resulting inserted ORF will then be aligned correctly for expression from the bacteriophage lambda pL promoter.

Because the cloned 1.9 kb *Bacillus subtilis* DNA did not contain an *Nde*I site at the desired location, a strategy was developed to introduce one at the ATG transcription start site of the *pnbA* ORF. The strategy involved separate constructions to generate amino-terminal and carboxy-terminal fragments of the *pnbA* gene, followed by ligation of the two fragments to a third fragment, obtained from the pHKY338 vector. Construction of the three fragments was accomplished as follows:

i) Amino-terminal fragment.

A 261 bp fragment coding for the amino-terminus of PNB esterase, containing an *Nde*I site adjacent and 5' to the ATG transcription start site, was synthesized using PCR technology (see Example 1E). The PCR amplification was performed using the following two primers listed from left to right in the 5' to 3' orientation:

```
1. PNBNDE1-AAAAAGGGAG AGAACCATAT GACTCATCAA ATAG (SEQ ID
NO.7)
```

```
2. PNB5-TTGACATACA AGCAATCCTC (SEQ ID NO. 8)
```

The PNBNDE1 sequence underlined above represents the ndel restriction site. The first three bases of the primer were not matched to the cloned PNB esterase gene. The other primer, PNB5, anneals approximately 25 base pairs (bp) downstream from the *Acc*I site located 215 bp downstream from the ATG start site. The 261 bp fragment that resulted from the amplification was treated with Proteinase K (protease Type XXVIII, from *Tritirachium album*) according to the procedure described by Crowe et *al.*, 1991, *Nuc. Acid Res.* 19:184, with minor modifications. The fragment was subsequently digested with *Nde*I and *Acc*I phenol extracted and ethanol precipitated by standard methods to generate the 180 bp fragment used in the ligation reaction below.

ii) Carboxy-terminal fragment

Since the construction strategy required use of the AccI site internal to the ORF to splice the amino-terminal and carrboxy-terminal fragments it was necessary to eliminate the other *Acc*I site (*Sal*I/*Acc*I) on the 1.9 kb fragment. This was accomplished in two steps: (i) digestion of pNBH3S1.9 with *Hind*III and *sal*I to release the ORF fragment; and (ii) ligation of the released fragment to pBluescript SK(-) that had been digested with *Hind*III and *Xho*I. Insertion of the *Sal*I/*Acc*I site of the 1.9 kb fragment into the *Xho*I site of pBluescript SK(-), eliminated all three sites.

Transformation of the ligation mixture into *Escherichia coli* K12 DH5α, followed by screening of putative clones and confirmation of structures by restriction digests, resulted in the isolation of pNBH3(SX)sk, which has only one *Acc*I site, located in the ORF. Another construction was needed to introduce a *Bam*HI site at the carboxy-terminus. This was accomplished by digestion of pNBH3(SX)sk with *Eco*RI and *Kpn*I, to release the ORF fragment, followed by ligation of this fragment into pUC19, also digested with *Eco*RI and *Kpn*I. Transformation of the ligation mixture into DH5α cells, followed by selection and analysis of clones, resulted in the isolation of plasmid pNBEKuc, which contained a single *Acc*I site in the ORF, and an appropriately positioned *Bam*HI site downstream from the carboxy-terminus.

iii) Vector fragment from pHKY338

Vector for the three piece ligation reaction described below, was prepared by digestion of pHKY338 with *Nde*I and *Bam*HI to remove the kanamycin resistance gene, and gel isolation of the large restriction fragment.

A three piece ligation was performed involving the *Nde*I-*Acc*I digested PCR fragment (180 bp), the *Acc*I-*Bam*HI fragment of pNBEKuc (1.7 kb), and the *Nde*I-*Bam*HI fragment of pHKY338 (6 kb). This produced plasmid vector pNB106R, containing the entire *pnbA* ORF under control of the temperature inducible lambda pL synthetic promoter. The resulting DNA was transformed into host strain *Escherichia coli* K12 DH5α. Plasmid pNB106R was subsequently transformed into host strains *E. coli* K12 RV308 and *E. coli* W ATCC 11105.

## EXAMPLE 4

Synthesis of PNB esterase by cultures of *Escherichia coli* K12 DH5α, *E. coli* K12 RV308 and *E. coli* W ATCC 11105 Transformed with Plasmid pNB106R

Synthesis of PNB esterase by cultures of *Escherichia coli* K12 DH5α, *E coli* K12 RV308 and *E coli* W ATCC 11105 transformed with plasmid pNB106R was induced as follows. A frozen stock culture of the strain was used to inoculate L-broth containing 5 μg/ml tetracycline. After 16 hr growth at 30°C, cells were subcultured (4% v/v) into fresh L-broth plus tetracycline and grown to mid-logarithmic phase. Induction of enzyme synthesis was accomplished by raising the temperature of the culture to 40°C. The kinetics of PNB esterase synthesis was measured by sampling the culture periodically and assaying enzyme activity in cell free extracts as described in Example 1.

Cell-free extracts were also analyzed by SDS-PAGE to allow the relative increase in the amount of PNB esterase protein to be monitored.

## EXAMPLE 5

Purification of PNB Esterase from Recombinant *Escherichia coli* Strains.

The PNB esterase was purified from *Escherichia coli* K12 DH5α/pNB106R by the following procedure which is outlined in Table 5. This procedure can be compared to the purification from *Bacillus*, presented in Example 1 and outlined in Table 4. It will be noted that in the latter case eight steps were required and 4.0 mg of pure enzyme was obtained from 41,400 mg of crude protein, whereas from the recombinant *Escherichia coli* strain 10 mg of pure enzyme was obtained from only 200 mg of crude protein using only six steps. Thus, the simpler process of purification, using recombinant *E. coli* as the source, was 3250 times more efficient than the old more complex purification process where the natural producer, *Bacillus subtilis*, was used as enzyme source.

Frozen cells (5.4 g) from *Escherichia coli* K12 DH5α/pNB106R, grown according to the method of Example 4, were thawed and homogenized in 20 ml of 10 mM potassium phosphate, pH 7.0, containing 0.5 mM EDTA and 1 mM β-mercaptoethanol (Buffer 1). The cell-free extract was obtained by centrifugation (28,000 x g, 15 min) of the cell homogenate and subsequently dialyzed overnight against 500 ml of 10 mM sodium acetate, pH 5.0. The dialysate was adjusted to pH 7.0 with 2 N NH$_4$OH and subjected to ammonium sulfate fractionation. The precipitates formed between 45 to 85% ammonium sulfate saturation was dissolved in 20 ml of 10 mM Tris-HCl, pH 8.5, containing 100 mM NaCl (Buffer 2) and dialyzed overnight against 500 ml of the same buffer. The dialysate was applied onto DE-52 column (1.6 x 20 cm) equilibrated with Buffer 2. The column was washed with 100 ml of Buffer 2 and 200 ml of 10 mM MES-NaOH, pH 6.5, containing 100 mM NaCl (Buffer 3), and the PNB esterase was eluted with a 375 ml of a linear gradient of 100 to 300 mM NaCl in Buffer 3. The fractions containing the esterase, which eluted between 180 to 210 mM NaCl, were pooled, concentrated to 20 ml by ultrafiltration (Amicon PM-30 membrane), and dialyzed overnight against 500 ml of 10 mM Tris-HCl, pH 8.0 (Buffer 4). The dialyzed esterase was applied onto a p-aminobenzamidine agarose column (1.0 x 20 cm) equilibrated with Buffer 4. After the column was washed with 50 ml of Buffer 4, the esterase was eluted from the column with 180 ml of a linear gradient of 0 to 300 mM NaCl in Buffer 4. The esterase, which eluted between 120 to 150 mM NaCl, was collected, concentrated to 15 ml by ultrafiltration, and dialyzed overnight against 500 ml of Buffer 2. The dialysate was applied onto a Q-Sepharose column (1.0 x 20 cm) equilibrated with Buffer 2. After washing the column with 50 ml of Buffer 2 followed by 50 ml of 10 mM MES-NaOH, pH 6.0, and 100 mM NaCl (Buffer 5), the esterase was eluted from the column with 150 ml of a linear gradient of 100 to 300 mM NaCl in Buffer 5. The esterase, which eluted between 180 to 210 mM NaCl, was collected and concentrated to 1 ml by ultrafiltration. The esterase preparation obtained was electrophoretically pure, as confirmed by SDS-PAGE. The purification data is summarized in Table 5.

Because the new purification procedure with recombinant *Escherichia coli* as the source of enzyme is considerably simpler, it would be less expensive to apply at large scale than the purification procedure needed for the same enzyme obtained from *B. subtilis*. This is a direct result of the significantly increased amount of PNB esterase obtained in the recombinant *E. coli* strain.

The PNB esterase purified from recombinant *Escherichia coli* was compared to the native enzyme by molecular weight, Western blot analysis, and substrate specificity.

Measurements of molecular weight gave values of 54,000 for enzyme from the two sources, and identical precipitin bands were obtained when the two enzyme preparations were tested using antibody prepared against native purified esterase. Both enzymes hydrolyzed cefaclor PNB ester, cefaclor nucleus PNB ester, loracarbef PNB ester, and loracarbef nucleus PNB ester. The native PNB esterase and the corresponding PNB esterase produced in recombinant *E. coli* strains appear functionally identical and structually the same at macroscale.

**Table 5**
**Purification of PNB esterase from *E. coli* K12 DH5α/pNB106R**

| Step | Protein (mg) | Activity to loracarbef nucleus (mUnits) | Activity of β-lactamase (mUnits) | Activity ratio of esterase/ β-lactamase | Sp. activity of esterase (mUnits/mg) | Fold | Yield (%) |
|---|---|---|---|---|---|---|---|
| cell free extract | 207.7 | 39658.7 | 669.3 | 59.3 | 190.9 | 1 | 100 |
| pH5 treatment | 188.5 | 41703.2 | 672.3 | 62.0 | 212.2 | 1.16 | |
| Ammonium sulfate fractionation (45%-85%) | 123.5 | 28200.0 | 488.5 | 57.7 | 228.3 | 1.2 | 71 |
| DE-52 | 33.30 | 24503.0 | 3.89 | 6299 | 735.8 | 3.85 | 62 |
| p-Aminobenzamidine agarose | 20.57 | 23813.2 | 0 | | 1157.6 | 6.06 | 60 |
| Q-sepharose | 10.08 | 13552.0 | 0 | | 1344.4 | 7.04 | 34 |

Starting cell mass = 5.4g wet weight
Substrate = loracarbef-nucleus-PNB ester

## Claims

1. *p*-Nitrobenzyl-esterase in substantially purified form.

2. The process for preparing the PNB esterase of Claim 1 in substantially pure form which comprises the steps:

   a) adding ammonium sulfate at about pH 7 to an aqueous cell-free extract containing PNB esterase to achieve a concentration of ammonium sulfate between about 45% and 80% of saturation, and collecting the resulting precipitate;

   b) dialyzing a solution of the precipitate buffered at about pH 7 in the presence of a reducing agent;

   c) acidifying the dialysate to about pH 5, removing the precipitates and adjusting the dialysate to about pH 8.5;

   d) chromatographing the dialysate over a weak anionic exchange resin and eluting the PNB esterase from said resin with a linear gradient of 0.05 M to 0.3 M NaCl in a buffer of about pH 7 in the presence of a reducing agent;

   e) pooling and concentrating the PNB esterase-containing eluate of step d;

   f) filtering the concentrate of step e through a polysaccharide-type gel at about pH 8.0, in the presence of a reducing agent;

   g) pooling and concentrating the PNB esterase-containing eluate of step f, and dialyzing a solution of the concentrate buffered at pH 8.5 in the presence of a reducing agent

   h) chromatographing the concentrate of step g over an anionic exchange resin at about pH 8.5 and eluting the PNB esterase from said resin with a linear gradient of 0.1 M to 0.3 M NaCl in a buffer of about pH 6, in the presence of a reducing agent;

   i) pooling and concentrating the PNB esterase-containing eluate of step h and dialyzing a solution of the concentrate buffered at about pH 5.0, in the presence of a reducing agent, and collecting the dialysate;

   j) chromatographing the dialysate over calcium phosphate-cellulose at about pH 6, and eluting the PNB esterase therefrom with a linear gradient of .01 to .10 M potassium phosphate at about pH 7, in the presence of a reducing agent;

   k) pooling and concentrating the PNB esterase-containing eluate of step j, dialyzing a solution of the concentrate buffered at about pH 8 in the presence of a reducing agent, and collecting the dialysate;

   l) chromatographing the dialysate over a *p*-aminobenzamidine-agarose at about pH 8, and eluting the PNB esterase therefrom with a linear gradient of 0 to 0.30 M NaCl at about pH 8, in the presence of a reducing agent;

   m) pooling and concentrating the PNB esterase-containing eluate of step 1;

   wherein steps a-m are carried out at a temperature between about 0°C and 4°C.

3. The process for preparing PNB esterase in substantially purified form from a recombinant host cell that is capable of expressing PNB esterase, comprising the steps:

   a) adding ammonium sulfate at about pH 7 to an aqueous cell-free extract containing PNB esterase to achieve a concentration of ammonium sulfate between about 45% and 85% of saturation, and collecting the resulting precipitate;

   b) dialyzing a solution of the precipitate buffered at about pH 7 in the presence of a reducing agent;

   c) acidifying the dialysate to about pH 5, removing the precipitates and adjusting the dialysate to about pH 8.5;

   d) chromatographing the dialysate over a weak anionic exchange resin and eluting the PNB esterase from said resin with a linear gradient of 0.05 M to 0.3 M NaCl in a buffer of about pH 7 in the presence of a reducing agent;

   e) pooling and concentrating the PNB esterase-containing eluate of step d;

   f) chromatographing the dialysate over a *p*-aminobenzamidine-agarose at about pH 8, and eluting the PNB esterase therefrom with a linear gradient of 0 to 0.3 M NaCl at about pH 8, in the presence of a reducing agent;

   g) pooling and concentrating the PNB esterase-containing eluate of step f, and dialyzing a solution of the concentrate buffered at pH 8.5 in the presence of a reducing agent

   h) chromatographing the concentrate of step g over an anionic exchange resin at about pH 8.5 and eluting the PNB esterase from said resin with a linear gradient of 0.1 M to 0.3 M NaCl in a buffer of about pH 6, in the presence of a reducing agent;

   i) pooling and concentrating the PNB esterase-containing eluate of step h and dialyzing a solution of the concentrate buffered at about pH 5.0, in the presence of a reducing agent, and collecting the dialysate.

4. A process for the removal of the *p*-nitrobenzyl group from a cephalosporin PNB ester or a 1-carbacephalosporin PNB ester which comprises reacting said ester with the PNB esterase of Claim 1.

5. The process of Claim 4 wherein the cephalosporin so produced cefaclor or loracarbef.

# FIG. I

FIG. 2

FIG. 3

European Patent Office   **EUROPEAN SEARCH REPORT**

Application Number

EP    92 31 1541

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | BIOCHEMICAL PHARMACOLOGY vol. 36, no. 14, 1987, OXFORD GB pages 2317 - 2324 CAMPBELL C J; CHANTRELL L J; EASTMOND R 'PURIFICATION AND PARTIAL CHARACTERIZATION OF RAT INTESTINAL CEFUROXIME AXETIL ESTERASE' * the whole document * | 1,4 | C12N9/18 C12P35/00 |
| Y | JOURNAL OF ANTIBIOTICS vol. 29, no. 2, 1976, TOKYO JP pages 121 - 124 Brannon, Donald R.; Mabe, James A.; Fukuda, David S. 'De-esterification of cephalosporin para- nitrobenzyl esters by microbial enzymes' * the whole document * | 1,4 | |
| D,A | US-A-3 972 774 (D.R.BRANNON) * the whole document * | 1,4 | |
| A | EP-A-0 403 172 (OKLAHOMA STATE UNIVERSITY) * abstract; claims 1-10 * | 2,3 | TECHNICAL FIELDS SEARCHED (Int. Cl.5 ) C12N C12P |
| A | EP-A-0 437 378 (GLAXO GROUP LTD) * claims 1-17 * | 3,4 | |
| A | EP-A-0 243 167 (SUMITOMO CHEMICAL COMPANY,LTD.) * example 3 * | 2,3 | |

The present search report has been drawn up for all claims

| Place of search | Date of completion of the search | Examiner |
|---|---|---|
| BERLIN | 16 MARCH 1993 | GURDJIAN D. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)

SEQUENCE LISTING

(1) GENERAL INFORMATION:
    (i)        APPLICANT:  Oklahoma State University
    (ii)       TITLE OF INVENTION:  PURIFIED *para*-NITROBENZYL
                    ESTERASE FROM *BACILLUS*
    (iii)      NUMBER OF SEQUENCES:  4
    (iv)       CORRESPONDENCE ADDRESS:
             (A)  ADDRESSEE:  Eli Lilly and Company
             (B)  STREET:  Erl Wood Manor
             (C)  CITY:  Windlesham
             (D)  STATE:  Surrey
             (E)  COUNTRY:  United Kingdom
             (F)  ZIP:  GU20 6PH
    (viii)     ATTORNEY/AGENT INFORMATION:
             (A)  NAME:  Hudson, Christopher Mark
             (B)  REGISTRATION NUMBER:  to follow
             (C)  REFERENCE/DOCKET NUMBER: X-8554
    (ix)       TELECOMMUNICATION INFORMATION:
             (A)  TELEPHONE: (0276) 478441
             (B)  TELEFAX: (0276) 478306


(2) INFORMATION FOR SEQ ID NO:1:
    (i) SEQUENCE CHARACTERISTICS:
        (A) LENGTH: 22 amino acids
        (B) TYPE: amino acid
        (D) TOPOLOGY: linear
    (ii) MOLECULE TYPE: peptide
    (xi) SEQUENCE DESCRIPTION: SEQ ID NO:1

```
Met Thr His Gln Ile Val Thr Thr Gln Tyr Gly Lys Val Lys Gly
 1               5                  10                  15
Thr Thr Glu Asn Gly Val His
            20
```

(2) INFORMATION FOR SEQ ID NO:2:
    (i) SEQUENCE CHARACTERISTICS:
        (A) LENGTH: 13 amino acids
        (B) TYPE: amino acid
        (D) TOPOLOGY: linear
    (ii) MOLECULE TYPE: peptide
    (xi) SEQUENCE DESCRIPTION: SEQ ID NO:2

```
Glu Asn Ile Phe Gln Leu Phe Phe Gln Pro Ala Leu Asp
 1               5                  10
```

(2) INFORMATION FOR SEQ ID NO:3:
    (i) SEQUENCE CHARACTERISTICS:
        (A) LENGTH: 22 amino acids
        (B) TYPE: amino acid
        (D) TOPOLOGY: linear
    (ii) MOLECULE TYPE: peptide
    (xi) SEQUENCE DESCRIPTION: SEQ ID NO:3

```
Ala Phe His Ala Leu Glu Leu Pro Phe Val Phe Gly Asn.Leu Asp
 1               5                      10                  15
Gly Leu Glu Xaa Met Ala. Lys
              20
```

(2) INFORMATION FOR SEQ ID NO:4:
    (i) SEQUENCE CHARACTERISTICS:
        (A) LENGTH: 15 amino acids
        (B) TYPE: amino acid
        (D) TOPOLOGY: linear
    (ii) MOLECULE TYPE: peptide
    (xi) SEQUENCE DESCRIPTION: SEQ ID NO:4

```
Gly Ile Pro Tyr Ala Lys Pro Pro Val Gly Gln Trp Trp Phe Lys
 1               5                      10                  15
```

(2) INFORMATION FOR SEQ ID NO:5:
    (i) SEQUENCE CHARACTERISTICS:
        (A) LENGTH:41 nucleotides
        (B) TYPE:nucleic acid
        (D) TOPOLOGY: linear
    (ii) MOLECULE TYPE:DNA
    (xi) SEQUENCE DESCRIPTION: SEQ ID NO:5

```
ATGACACATC AAATTGTCAC AACATATGGC AAAAAAGTCA A 41
```

2) INFORMATION FOR SEQ ID NO:6:
    (i) SEQUENCE CHARACTERISTICS:
        (A) LENGTH:41 nucleotides
        (B) TYPE:nucleic acid
        (D) TOPOLOGY: linear
    (ii) MOLECULE TYPE:DNA 6
    (xi) SEQUENCE DESCRIPTION: SEQ ID NO:6

```
TATGGCAAAA AAGTCAAAGG CACACAAGAA AATGGCGTCC A 41
```

    (2) INFORMATION FOR SEQ ID NO:7:
        (i) SEQUENCE CHARACTERISTICS:
             (A) LENGTH:34 nucleotides
             (B) TYPE:nucleic acid
             (D) TOPOLOGY:linear
        (ii) MOLECULE TYPE:DNA
        (xi) SEQUENCE DESCRIPTION:SEQ ID NO:7

```
AAAAAGGGAG AGAACCATAT GACTCATCAA ATAG
```

    (2) INFORMATION FOR SEQ ID NO:8:
        (i) SEQUENCE CHARACTERISTICS:
             (A) LENGTH:20 nucleotides
             (B) TYPE:nucleic acid
             (D) TOPOLOGY:linear
        (ii) MOLECULE TYPE:DNA
        (xi) SEQUENCE DESCRIPTION:SEQ ID NO:8

```
TTGACATACA AGCAATCCTC 20
```